(19) Europäisches Patentamt
European Patent Office
Office européen des brevets

(11) EP 2 550 964 A1

(12) # EUROPEAN PATENT APPLICATION

(43) Date of publication:
**30.01.2013 Bulletin 2013/05**

(21) Application number: **11175435.4**

(22) Date of filing: **26.07.2011**

(51) Int Cl.:
***A61K 31/355*** *(2006.01)* ***A61K 31/436*** *(2006.01)*
*A61P 25/22* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Universitat Pompeu-Fabra**
**08002 Barcelona (ES)**

(72) Inventors:
- **Ozaita Mintegui, Andrés**
  **E-08003 Barcelona (ES)**
- **Puighermanal Puigvert, Emma**
  **E-08003 Barcelona (ES)**
- **Busquets Garcia, Arnau**
  **E-08003 Barcelona (ES)**
- **Maldonado Lopez, Rafael**
  **E-08003 Barcelona (ES)**

(74) Representative: **ABG Patentes, S.L.**
**Avenida de Burgos, 16D**
**Edificio Euromor**
**28036 Madrid (ES)**

(54) **Temsirolimus for use in the prevention and/or treatment of a delta 9-tetrahydrocannabinol-induced anxiety disorder**

(57) The invention relates to temsirolimus for use as a medicament to prevent and/or treat a Δ9-tetrahydrocannabinol-induced anxiety disorder and to compositions comprising both agents.

EP 2 550 964 A1

## Description

### FIELD OF THE INVENTION

The invention relates to temsirolimus for use as a medicament to prevent and/or treat a Δ9-tetrahydrocannabinol-induced anxiety disorder and to compositions comprising both temsirolimus and Δ9-tetrahydrocannabinol.

### BACKGROUND OF THE INVENTION

Δ9-tetrahydrocannabinol (Δ9-THC), also known as tetrahydrocannabinol (THC) is the main psychoactive substance found in the cannabis plant. Dronabinol is the name of a pure isomer of THC, (-)-trans-Δ9-tetrahydrocannabinol, that is the main isomer in cannabis.

THC provides medical benefits for cancer and AIDS patients by increasing appetite and decreasing nausea. It has also been shown to assist some glaucoma patients by reducing pressure within the eye and is used in the form of cannabis by a number of multiple sclerosis patients, who use it to alleviate neuropathic pain and spasticity.

THC is also an experimental therapy for cancer-induced cachexia (Gullett N. et al. 2009. J Soc Integr Oncol 7 (4): 155-169).

However, the administration of THC has several negative side-effects. One of the most common side effects is THC-induced anxiety disorder (Crippa J.A. et al. 2009. Hum Psychopharmacol 24(7): 515-23; Guillem E. et al.2009. Encephale 35(3): 226-233; Leweke F.M. and Koethe D. 2008. Addict Biol 13(2): 264-275; Williamson E.M. and Evans F.J.2000. Drugs 60(6):1303-1314) which can limit the use of said drug.

There is no specific treatment for THC-induced anxiety disorder.

Thus, it is highly desirable to provide new therapeutic agents for the prevention and/or treatment of said disorder.

### SUMMARY OF THE INVENTION

In one aspect, the invention relates to temsirolimus for use as a medicament in the prevention and/or treatment of a Δ9-tetrahydrocannabinol-induced anxiety disorder in a mammal.

In another aspect, the invention relates to a pharmaceutical composition comprising:

(i) a pharmaceutically effective amount of temsirolimus,

(ii) a pharmaceutically effective amount of Δ9-tetrahydrocannabinol; and

(iii) at least one pharmaceutically acceptable excipient.

### BRIEF DESCRIPTION OF THE FIGURES

**Figure 1.** Graphic showing the cognitive capacity measured by the object-recognition test. ***p<0.001 compared to the vehicle group.

**Figure 2.** Blockade of the amnesic effects of THC produced by the administration of temsirolimus measured by the object-recognition test. The pre-treatment with temsirolimus avoids the amnesic effects of THC. ***p<0.001 compared to the vehicle group; ###p<0.001 compared to the THC 10 group.

**Figure 3.** The chronic administration of THC 10 mg/kg/day (30 mg/$m^2$/day), once a day, produces a cognitive deficit that can be assessed by the object-recognition test. **A:** After finishing the exposition to THC (from day 6), the animal continues showing a cognitive deficit during the following four days. **B:** The administration of temsirolimus 1 mg/kg/day (3 mg/$m^2$/day) together with THC 10 mg/kg/day (30 mg/$m^2$/day) prevented the appearance of the THC cognitive deficit. ***p<0.001, **p<0.01, *p<0.05 compared to the vehicle group.

**Figure 4.** Blockade of the anxiogenic effect of THC 10 mg/kg/day (30 mg/$m^2$/day) produced by the administration of temsirolimus measured by the elevated plus maze test. ***p<0.001 compared to the vehicle group; ###p<0.001 compared to the THC 10 group.

**Figure 5.** Temsirolimus 1 mg/kg/day (3 mg/$m^2$/day) does not avoid the anxiolytic effect of the THC administered at

a dose of 0.3 mg/kg/day (0.9 $mg/m^2$/day) measured by the elevated plus maze test. ***$p<0.001$ compared to the vehicle group.

**Figure 6.** Temsirolimus 1 mg/kg/day (3 mg/kg/day) does not avoid the analgesic effect showed by THC 10 mg/kg/day (30 $mg/m^2$/day) measured by the visceral pain test. ***$p<0.001$ compared to the vehicle group.

## DETAILED DESCRIPTION OF THE INVENTION

Inventors have surprisingly found that the administration of temsirolimus prevents the negative effects produced by the psychoactive component of cannabis Δ9-tetrahydrocannabinol (THC) such as the acute or chronic cognitive deficit and has an unexpected and advantageous effect by blocking the anxiogenic effect of THC, whereas it does not alter the analgesic and anxiolytic beneficial effects of THC. Thus, temsirolimus is an agent useful in the prevention and/or the treatment of a Δ9-tetrahydrocannabinol-induced anxiety disorder without altering the therapeutic activity of THC.

Temsirolimus is a rapamycin derivative capable of specifically inhibiting mTOR (mammalian target of rapamycin) enzyme. mTOR is a serine/threonine protein kinase that regulates cell growth, cell proliferation, cell motility, cell survival, protein synthesis, and transcription (Hay N. and Sonenberg N. 2004. Genes Dev, 18(16): 1926-45). This invention relates to the use of temsirolimus in preventing and/or treating a Δ9-tetrahydrocannabinol-induced anxiety disorder but this could be applied to rapamycin (sirolimus) and all the family of rapamycin derivatives such as RAD-001 (everolimus) or AP23573.

## THERAPEUTIC USES OF THE INVENTION

Therefore, a first aspect of the invention relates to temsirolimus for use as a medicament in the prevention and/or treatment of a Δ9-tetrahydrocannabinol-induced anxiety disorder in a mammal.

In another aspect, the invention relates to the use of temsirolimus for the preparation of a medicament for the prevention and/or the treatment of a Δ9-tetrahydrocannabinol-induced anxiety disorder in a mammal.

In another aspect, the invention relates to a method of prevention and/or treatment of a mammal suffering from a Δ9-tetrahydrocannabinol-induced anxiety disorder comprising the administration of temsirolimus.

Temsirolimus, also named CCI-779, is a derivative of sirolimus that specifically inhibits mammalian target of rapamycin (mTOR) protein and interferes with the synthesis of proteins that regulate proliferation, growth and survival of tumor cells. Temsirolimus is used as intravenous drug for the treatment of renal cell carcinoma.

The chemical name of temsirolimus is (1R,2R,4S)-4-{(2R)-2-[(3S,6R,7E,9R,10R,12R,145,15E,17E,19E, 215,23S,26R,27R,34aS)-9,27-dihydroxy-10,21-dimethoxy-6,8,12,14,20,26-hexamethyl-1,5,11,28,29-pentaoxo-1,4,5,6,9,10,11,12,13,14,21,22,23,24,25,26,27,28,29,31,32,33,34,34a-tetracosahydro-3H-23,27-epoxypyrido[2,1-c] [1,4]oxazacyclohentriacontin-3-yl]propyl}-2-methoxycyclohexyl 3-hydroxy-2-(hydroxymethyl)-2-methylpropanoate and its chemical structure is represented by:

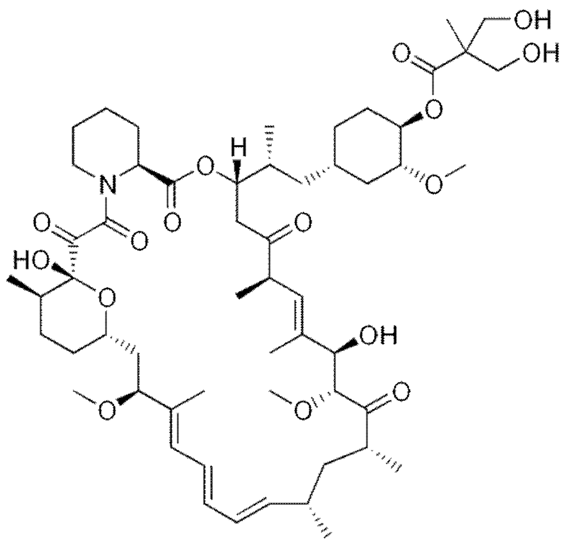

The term "medicament" refers to a pharmaceutical composition comprising temsirolimus. The medicament may be administered orally, by injection, by inhalation or insuflation or by the rectal route All these pharmaceutical compositions

are prepared by conventional means with pharmaceutically acceptable excipients. Oral and parenteral formulations are preferred.

In one embodiment of the present invention the medicament is prepared for oral administration. Pharmaceutical compositions for oral administration are in any suitable dosage form such as syrups, solutions, suspensions, tablets, capsules, lozenges, controlled-release preparations, fast-dissolving preparations or they may be presented as a dry product for reconstitution with water or other suitable vehicle before use.

Pharmaceutical compositions for injection (subcutaneous, intradermic, intramuscular, intravenous, etc.) including suspensions, solutions, emulsions in oily or aqueous vehicles, pastes and implantable sustained-release or biodegradable formulations. Such formulations can further comprise one or more additional ingredients including suspending, stabilizing and/or dispersing agents. In one embodiment of a formulation for parenteral administration, the active ingredient is provided in dry (i. e. powder or granular) form for reconstitution with a suitable vehicle (e. g. sterile pyrogen-free water) prior to parenteral administration of the reconstituted composition.

Other parentally-administrable formulations which are useful include those which comprise the active ingredient in microcrystalline form, in a liposomal preparation, or as a component of a biodegradable polymer system. Compositions for sustained release or implantation can comprise pharmaceutically acceptable polymeric or hydrophobic materials such as an emulsion, an ion exchange resin, a sparingly soluble polymer, or a sparingly soluble salt.

The pharmaceutical formulations may conveniently be presented in unit dosage form and may be prepared by any of the methods well known in the art of pharmacy. All methods include the step of bringing the active ingredient(s) association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A syrup formulation will generally consist on a suspension or solution of the compound or salt in a liquid carrier for example natural, synthetic or semisynthetic oils or water with flavouring, sweetener and/or colouring agent.

When the composition is in the form of a tablet, any pharmaceutical carrier routinely used for preparing solid formulations may be used.

A tablet may be made by compression or moulding, optionally with one or more accessory ingredients. Compressed tablets may be prepared by compressing in a suitable machine the active ingredient in a free-flowing form such as a powder or granules, optionally mixed with binders, lubricants, inert diluents, surface active or dispersing agents. Moulded tablets may be made by moulding in a suitable machine a mixture of the powdered blend comprising the active compounds moistened with an inert liquid diluent and optionally dried and sieved. The tablets may optionally be coated or scored and may be formulated so as to provide modified (i. e. slow or controlled) release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered.

Formulations for injection may be presented in unit dosage form (e.g. in ampoules) or in multidose containers with an added preservative.

"Prevention" is understood as the administration of temsirolimus or of a medicament containing it in an initial or early stage of the THC-induced anxiety disorder, or to also prevent its onset.

The term "treatment" is used to designate the administration of temsirolimus or compositions thereof to control disorder progression before or after the clinical signs had appeared. By control of the disorder progression it is meant to designate beneficial or desired clinical results including, but not limited to, reduction of symptoms, reduction of the length of the THC-induced anxiety disorder, stabilization pathological state (specifically avoidance of further deterioration), delay in the disorder's progression, improvement of the pathological state and remission (both partial and total). In a particular embodiment of the invention temsirolimus is used to control the disorder progression once at least one of the disorder's clinical signs has appeared.

The term "Δ9-tetrahydrocannabinol" refers to the compound also named tetrahydrocannabinol (Δ9-THC or THC) or dronabinol. It is an aromatic terpenoid, having very low solubility in water, but good solubility in most organic solvents.

THC is a psychoactive substance found in the cannabis plant. Thus, it can be isolated from female plants pertaining to the genus *Cannabis. Cannabis* is a genus of flowering plants that includes three putative species: *Cannavis sativa*, *Cannabis indica* and *Cannabis ruderalis. Cannabis ruderalis* has a lower THC content than either *Cannabis sativa* or *Cannabis indica.* However, *C. indica* strains are frequently cross-bred with *C. ruderalis* to produce plants combining a higher THC content with the hardiness and reduced height of *C. ruderalis.* In the cannabis plant, THC occurs mainly as tetrahydrocannabinol carboxylic acid (THC-COOH). Over time, or when heated, THC-COOH is decarboxylated producing THC. The psychoactive product consists of dried flowers of plants selectively bred to produce high levels of THC and other psychoactive chemicals.

Δ9-THC may be administered in the form of pharmaceutical compositions comprising it as sole active ingredient or in combinations with other active ingredients. There are also marketed drugs obtained from an extract of Cannabis plant, such as Sativex®, that contains a standardised composition, formulation and dose comprising tetrahydrocannabinol (THC) and cannabidiol (CBD). Sativex® is the trade name of a cannabinoid mouth spray for multiple sclerosis patients who can use it to alleviate neuropathic pain, spasticity, overactive bladder and other symptoms. The product is formulated as an oromucosal spray which is administered by spraying into the mouth. Sativex® is approved in Canada, United Kingdom, New Zealand, the Czech Republic, Spain, Germany and Denmark. In some countries, such as Canada, it has also been approved as a potential treatment to alleviate pain due to cancer and in other countries is under clinical trials for said use. Sativex® has also been recommended for approval in Italy, Sweden and Austria with formal approvals expected in these countries during 2011.

Alternatively, it may also be administered as medical cannabis also known as medical marijuana, which is the use of cannabis and its constituent cannabinoids such as THC as a physician-recommended form of medicine or herbal therapy. Medical marijuana contains more than 60 cannabinoids including THC. Although the extent of the medicinal value of cannabis has been disputed, it does have several well-documented beneficial effects. Among these are: the amelioration of nausea and vomiting, stimulation of hunger in chemotherapy and AIDS patients, lowered intraocular eye pressure (shown to be effective for treating glaucoma), as well as gastrointestinal illness. Its effectiveness as an analgesic has been suggested and disputed as well. There are several methods for administration of dosage, including vaporizing or smoking dried buds, drinking, or eating extracts, and taking capsules. The use of cannabis as a medicine is legal in a number of territories, including Canada, Austria, Germany, the Netherlands, Spain, Israel, Italy, Finland and Portugal.

Thus, in an embodiment the Δ9-tetrahydrocannabinol is contained in a plant of the genus *Cannabis,* preferably selected from the group *Cannabis sativa*, *Cannabis indica*, *Cannabis ruderalis*, hybrids thereof and mixtures thereof; more preferably *Cannabis sativa.*

The term "hybrid" referred to *Cannabis* species is meant plants obtained by crossing different species of the Genus *Cannabis.* The invention also contemplates mixtures of said different species.

THC can also be obtained by chemical synthesis. Synthetic cannabinoids are available as prescription drugs in some countries. The International Non-propietary name of the pure isomer of THC, (-)-trans-Δ9-tetrahydrocannabinol, is dronabinol. This is the main isomer in cannabis. Its pharmacological actions result from its binding to the cannabinoid receptor CB1, located mainly in the central nervous system, and the CB2 receptor, mainly present in cells of the immune system. Dronabinol is available as a prescription drug in several countries including the United States and Germany under the name Marinol™. Marinol™ has been approved by the FDA in the treatment of anorexia in AIDS patients, as well as for refractory nausea and vomiting of patients undergoing chemotherapy. Dronabinol is administered in oral form or by inhalation.

The chemical name of tetrahydrocannabinol (THC) or dronabinol is (-)-(6aR,10aR)-6,6,9-trimethyl-3-pentyl-6a, 7,8,10a-tetrahydro-6H-benzo[c]chromen-1-ol and its chemical structure is represented by:

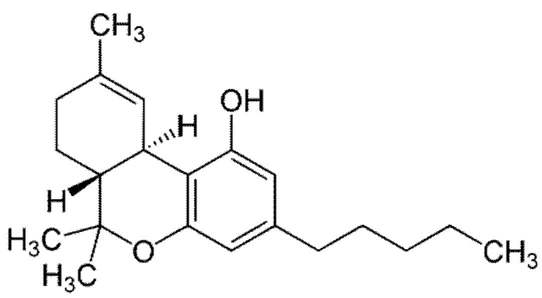

THC has mild to moderate analgesic effects and both synthetic and natural as a component of Cannabis can be used to treat pain. However, it is associated to other effects such as relaxation; euphoria; altered space-time perception; alteration of visual, auditory and olfactory senses; anxiety; disorientation; fatigue; and appetite stimulation. It also has anti-emetic properties. There are studies that indicate that THC has an anticholinesterase action (Brown H. 1972. Psychopharmacologia 27(2): 11-16; Eubanks LM. et al. 2006. Molecular Pharmaceutics 3(6):773-777) which may implicate it as a potential treatment for Alzheimer's and Myasthenia Gravis.

One of the negative side effects of THC at high doses (above 9 $mg/m^2/day$) is the induction of anxiety (reviewed in Moreira F.A. and Wotjak C.T. 2010. Curr Top Behav Neurosci 2:429-450). Said anxiogenic effect is not shown at low doses (for example at 0.9 $mg/m^2/day$) since low doses of THC produce anxiolytic effects.

Thus, the term "Δ9-tetrahydrocannabinol" or "THC" in the present invention includes synthetic THC such as

dronabinol and also the active ingredient THC found in naturally occurring plants of the genus *Cannabis,* such as the administration of an extract of marijuana (Sativex®) or the herbal plant. In a preferred embodiment the THC is synthetic THC, particularly dronabinol.

The term "THC" or "Δ9-tetrahydrocannabinol" includes pharmaceutically acceptable salts thereof.

The term "pharmaceutically acceptable" refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

The term "pharmaceutically acceptable salt" embraces salts with a pharmaceutically acceptable acid or base. Pharmaceutically acceptable acids include both inorganic acids, for example and without limitation hydrochloric, sulfuric, phosphoric, diphosphoric, hydrobromic, hydroiodic and nitric acid and organic acids, for example and without limitation citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example and without limitation alkyl amines, arylalkyl amines and heterocyclic amines.

In the present invention, the expression "Δ9-tetrahydrocannabinol-induced anxiety disorder" refers to an anxiety disorder caused by the compound Δ9-tetrahydrocannabinol or a product containing it. The THC-induced anxiety disorder is distinguished from a primary anxiety disorder by the fact that THC is judged to be etiologically related to the symptoms. Thus, the definition of the "Δ9-tetrahydrocannabinol-induced anxiety disorder" of the present invention does not include anxiety disorders due to other causes than the administration of said compound.

The term "mammal" includes, but is not restricted to, domestic and farm mammals, primates and humans, e.g., human beings, non-human primates, cows, horses, pigs, sheep, goats, dogs, cats or rodents. In a preferred embodiment the mammal is a human. The authors of the present invention have found that temsirolimus blocks the anxiogenic effect of high doses of THC in mice by using the elevated plus maze test described in the Examples section. According to the present invention, temsirolimus is found to be effective in blocking the anxiogenic effect of Δ9-tetrahydrocannabinol at doses which do not produce amnesic effects and do not have effect over the recognition memory.

By "amnesic effects" is understood the condition in which memory is disturbed or lost.

Thus, in a preferred embodiment temsirolimus is administered at a dose that does not have amnesic effects. Said dose can be determined by the skilled in the art by the object-recognition test as described in the Examples section. In a more preferred embodiment the medicament is prepared for the administration of a daily dose of temsirolimus not having amnesic effects, preferably from 1.5 $mg/m^2$ to 15 $mg/m^2$, more preferably 3 $mg/m^2$.

In an embodiment of the present invention the medicament comprises temsirolimus as a sole therapeutic agent. However, it may be advantageous to combine the administration of A9-tetrahydrocannabinol with temsirolimus. Thus, in another embodiment of the present invention the medicament comprises temsirolimus and Δ9-tetrahydrocannabinol.

Low doses of Δ9-tetrahydrocannabinol do not produce anxiogenic effects. Thus, the Δ9-tetrahydrocannabinol of the present invention is administered at high doses. In a preferred embodiment the medicament comprises temsirolimus and more than 9 $mg/m^2$/day of Δ9-tetrahydrocannabinol. In a more preferred embodiment the Δ9-tetrahydrocannabinol is administered as a daily dose of 30 $mg/m^2$.

By "combined administration" it has to be understood that temsirolimus can be administered together or separately, simultaneously, concurrently or sequentially with Δ9-tetrahydrocannabinol in any order, e.g. the administration of temsirolimus can be made first, followed by the administration of Δ9-tetrahydrocannabinol; or the administration of temsirolimus can be made last, preceded by the administration of Δ9-tetrahydrocannabinol; or the administration of temsirolimus can be made concomitantly with Δ9-tetrahydrocannabinol.

Thus, in a preferred embodiment temsirolimus is administered before the administration of Δ9-tetrahydrocannabinol. In another preferred embodiment temsirolimus is administered concomitantly with Δ9-tetrahydrocannabinol.

A person skilled in the art understands, in the context of the present invention, that the medicament for combined administration of temsirolimus and Δ9-tetrahydrocannabinol can be in the form of a single dosage form or in separate dosage forms. In an embodiment of the present invention, temsirolimus and Δ9-tetrahydrocannabinol are contained in separate dosage forms.

In another embodiment of the present invention temsirolimus and Δ9-tetrahydrocannabinol are contained in a single dosage form.

When temsirolimus is used for the treatment of a human patient the amount of temsirolimus to be administered ranges from 0.04 mg/kg/day to 0.41 mg/kg/day, preferably 0.08 mg/kg/day. These doses, in the case of a patient having an average body weight of 60 Kg, correspond to daily doses ranging from 2.4 mg/day to 24.6 mg/day, preferably 4.8 mg/day.

PHARMACEUTICAL COMPOSITIONS

Another aspect of the present invention is a pharmaceutical composition comprising:

(i) a pharmaceutically effective amount of temsirolimus,
(ii) a pharmaceutically effective amount of Δ9-tetrahydrocannabinol; and
(iii) at least one pharmaceutically acceptable excipient.

In the present invention, the term "pharmaceutical composition" relates to a formulation that has been adapted for administering a predetermined dose of two or more therapeutic useful agents to a cell, a group of cells, an organ, a tissue or an animal in need thereof. Said pharmaceutical composition comprises a single dosage form or separate dosage forms for components (i) and (ii).

The terms "temsirolimus" and "Δ9-tetrahydrocannabinol" have been defined previously in the context of the therapeutic uses of the invention.

The "pharmaceutically effective amount of temsirolimus" is an amount of said drug capable of blocking the anxiogenic effect of Δ9-tetrahydrocannabinol and not having amnesic effects. Said dose can be determined by the skilled in the art by the object-recognition test as described in the Examples section.

In a preferred embodiment the pharmaceutically effective amount of temsirolimus ranges from 1.5 $mg/m^2$ to 15 $mg/m^2$, more preferably 3 $mg/m^2$.

When temsirolimus is used for the treatment of a human patient it will be administered in quantities ranging from 0.04 mg/kg/day to 0.41 mg/kg/day; preferably 0.08 mg/kg/day. These doses, in the case of a patient having an average body weight of 60 Kg, correspond to daily doses of from 2.4 mg/day to 24.6 mg/day, preferably 4.8 mg/day.

The "pharmaceutically effective amount of Δ9-tetrahydrocannabinol" is the amount of said drug capable of having therapeutic effects. Said amount may vary depending on the disorder or disease to be treated and can be determined by the person skilled in the art by commonly used means. Low doses of Δ9-tetrahydrocannabinol (for example 0.9 $mg/m^2$/day) do not have anxiogenic effects. Thus, the amount of Δ9-tetrahydrocannabinol is preferably a high dose having anxiogenic effects. Tipically, Δ9-tetrahydrocannabinol is administered as a daily dose of 9 $mg/m^2$/day to 90 $mg/m^2$/day, more preferably 30 $mg/m^2$/day.

The "pharmaceutically effective amount of Δ9-tetrahydrocannabinol" includes a pharmaceutically effective amount of salts thereof.

When Δ9-tetrahydrocannabinol is used for the treatment of a human patient, it will be administered in quantities ranging from 0.24 mg/kg/day to 2.43 mg/kg/day; preferably 0.81 mg/kg/day. These doses, in the case of a patient having an average body weight of 60 Kg, correspond to daily doses of from 14.40 mg to 145.80 mg, preferably 48.60 mg.

In a preferred embodiment the Δ9-tetrahydrocannabinol is contained in a plant of the genus *Cannabis,* preferably selected from the group *Cannabis sativa*, *Cannabis indica, Cannabis ruderalis*, hybrids thereof and mixtures thereof; preferably *Cannabis sativa.*

These daily doses are preferably administered once a day. The content of temsirolimus and Δ9-tetrahydrocannabinol in the pharmaceutical composition will vary according to the number of daily administrations.

Thus, according to one embodiment of the present invention the pharmaceutical composition is formulated for the administration once a day and will contain from 2.40 to 24.60 mg of temsirolimus and from 14.40 to 145.80 mg of Δ9-tetrahydrocannabinol, preferably 4.80 mg of temsirolimus and 48.60 mg of Δ9-tetrahydrocannabinol.

The pharmaceutical composition of the invention is prepared by conventional means with one or more pharmaceutically acceptable excipients.

"Pharmaceutically acceptable excipient" is understood a therapeutically inactive substance said to be used for incorporating the active ingredient(s) and which is acceptable for the patient from a pharmacological/toxicological point of view and for the pharmaceutical chemist who manufactures it from a physical/chemical point of view with respect to the composition, formulation, stability, acceptation of the patient and bioavailability.

The number and the nature of the pharmaceutically acceptable excipients depend on the desired dosage form. The pharmaceutically acceptable excipients are known by the person skilled in the art (Fauli y Trillo C. (1993) "Tratado de Farmacia Galénica", Luzán 5, S.A. Ediciones, Madrid). Said compositions can be prepared by means of the conventional methods known in the state of the art ("Remington: The Science and Practice of Pharmacy", 20th edition (2003) Genaro A.R., ed., Lippincott Williams & Wilkins, Philadelphia, US).

Said pharmaceutical compositions can be administered by any type of suitable route, such as by oral route, by inhalation or parenteral route so that the pharmaceutically acceptable excipients necessary for the formulation of the desired dosage form will be included.

The components (i) and (ii) can be in two independent pharmaceutical compositions forming a kit of parts. This is particularly useful when components (i) and (ii) are to be administered by different routes. Thus, in a preferred embodiment, components (i) and (ii) of the composition are contained in separate dosage forms.

Alternatively, in another preferred embodiment, components (i) and (ii) of the composition are contained in a single dosage form.

The preferred routes of administration of the pharmaceutical composition are the parenteral and oral route. In a more preferred embodiment the route of administration is the oral route.

"Oral route" is understood as the pharmaceutical composition incorporated into the organism after deglutition. In a particular embodiment, the pharmaceutical composition of the invention can be in a dosage form suitable for its administration by oral route, whether it is solid or liquid. The dosage forms suitable for their administration by oral route can be tablets, capsules, syrups or solutions, and can contain any conventional excipient known in the art, such as binders, for example syrup, acacia, gelatin, sorbitol or polyvinylpyrrolidone; filling agents, for example lactose, sugar, corn starch, calcium

phosphate, sorbitol or glycine; lubricants for compression, for example, magnesium stearate; disintegrating agents, for example starch, polyvinylpyrrolidone, sodium glycolate of starch or microcrystalline cellulose; or pharmaceutically acceptable wetting agents such as sodium lauryl sulfate. The solid oral compositions can be prepared by means of conventional processes of mixing, filling or compressing. Repetitive mixing operations can be used to completely distribute the active agent in those compositions that use high amounts of filling agents. Said operations are conventional in the art. The tablets can be prepared, for example, by means of wet or dry granulation, and optionally coating them according to the processes known in the common pharmaceutical practice, particularly with an enteric coating.

As it is used herein, the term "parenteral", includes administration by intravenous route, intraperitoneal route, intramuscular route or subcutaneous route. Other than the oral route, intravenous and subcutaneous dosage forms of parenteral administration are generally preferred, being subcutaneous dosage forms more preferred.

In one embodiment, the pharmaceutical compositions of the invention can be adapted for their parenteral administration, such as sterile solutions, suspensions or lyophilized products in the appropriate dosage unit form. The pharmaceutical compositions suitable for its injectable use include sterile aqueous solutions (when they are soluble in water), or dispersions and sterile powders for the extemporaneous preparation of sterile injectable solutions or dispersions. For its administration by intravenous route, some suitable carriers include saline solution buffered with phosphate (PBS). In all the cases, the composition must be sterile, and must be fluid to the point of which there exists easy ability to inject. It must be stable in the preparation and storage conditions, and must be protected from the contamination action of microorganisms such as bacteria and fungi. The carrier can be a solvent or a dispersion medium which contains, for example, water, ethanol, a pharmaceutically acceptable polyol such as glycerol, propylene glycol, liquid polyethylene glycol and suitable mixtures thereof. Suitable fluidity can be maintained, for example, by means of using a coating such as lecithin, by means of maintaining the particle size required in the case of a dispersion and by means of using surfactants. The prevention of the action of the microorganisms can be achieved by means of various antibacterial and antifungal agents, for example, parabens, chlorobutanol, phenol, ascorbic acid, thiomersal, and the like. In most cases, it will be preferable to include isotonic agents, for example, sugars; polyalcohols such as mannitol, sorbitol; or sodium chloride in the composition. The prolonged absorption of the injectable compositions may be caused by the inclusion of an agent which delays the absorption, for example, aluminum and gelatin monostearate.

The injectable sterile solutions can be prepared by incorporating the active compound in the required amount in a suitable solvent with one or a combination of the aforementioned ingredients, as needed, followed by sterilization by filtration through sterile membranes. Generally, the dispersions are prepared by incorporating the active compound in a sterile vehicle containing a basic dispersion medium and the rest of the ingredients required from among those previously listed. In the case of sterile powders for the preparation of injectable sterile solutions, the preferred preparation processes are vacuum drying and lyophilization which give rise to a powder with the active ingredient plus any desired additional ingredient from a previously filtered sterile solution thereof.

The compositions of the invention are suitable for the administration into any type of mammal, preferably a human being.

The embodiments disclosed in the context of the therapeutic uses of the invention are also applicable to the compositions of the invention.

## EXAMPLES

Experiments in the present invention, performed in mice, had shown that temsirolimus is effective at a daily dose of 3 $mg/m^2$/day to prevent and/or treat the anxiogenic effect of THC.

Doses of temsirolimus may be expressed either in mg of temsirolimus per kg of body weight or in mg of temsirolimus per square meter of body surface. The article from Reagan-Shaw S. "Dose translation from animal to human studies revisited". FASEB J 2007, 22:659-661 provides the standard conversion factors used to convert mg/kg to $mg/m^2$.

$$\text{Dose (mg/kg)} \times K_m = \text{Dose } (mg/m^2)$$

The article also explains that this conversion is the basis for converting dose in a first animal species to dose

in a second animal species (allometric dose translation). Thus, animal dose (AD) in mg/kg can be converted to human equivalent dose (HED) in mg/kg using the following formula:

$$HED\ (mg/kg) = AD\ (mg/kg) \times \frac{\text{Animal } K_m}{\text{Human } K_m}$$

wherein the $K_m$ for each species is shown in Table I (data extracted from Reagan-Shaw S. Dose translation from animal to human studies revisited. FASEB J 2007, 22:659-661).

**Table I. $K_m$ factor for conversion of AD to HED**

| Species | | $K_m$ factor |
|---|---|---|
| Human | Adult | 37 |
| | Child | 25 |
| Baboon | | 20 |
| Dog | | 20 |
| Monkey | | 12 |
| Rabbit | | 12 |
| Guinea pig | | 8 |
| Rat | | 6 |
| Hamster | | 5 |
| Mouse | | 3 |

Thus, the experiment with a dose of 1 mg/kg/day in mice correspond to general doses in mammals of 3 $mg/m^2$/day.

The advantages of the invention are more fully illustrated with reference to the following examples.

## Material and Methods

### Object-recognition task

Object-recognition memory was assayed in the V-maze, a black Plexiglas maze with two corridors (30 cm long x 4.5 cm wide) set in V with a 90 degrees angle, and 15 cm high walls. The test was performed as previously described (Puighermanal, E. et al., 2009. Nature Neuroscience, 12:1152-1158). Briefly, a discrimination index was calculated in the test session. All the drugs (temsirolimus or THC and their vehicles) were injected intraperitoneally after the training session. Objects to be explored in the test were made out of different materials (marble, wood or plastic) and shapes, and had been tested in previous experiments to show no preference or dislike for control mice. On the first day, mice were placed for 9 min in the V-maze for habituation. On the second day, mice were placed back in the V-maze where two objects were positioned at the end of the corridors. On the next day (test day), mice were placed in the V-maze where one of the objects was replaced by a new object. The time spent exploring the familiar object (Tf) and the novel object (Tn) were counted and the discrimination index (D.I.) was calculated as follows: D.I.= [Tn-Tf]/[Tn+Tf]. D.I. close to zero means a cognitive deficit, while D.I. close to 0.5 means a good cognitive function in this memory test.

### Anxiolytic-like responses

*Elevated plus maze test.* The elevated plus maze test was performed in a black Plexiglas apparatus with four arms (29 cm long x 5 cm wide) set in cross from a neutral central square (5 cm x 5 cm) elevated 40 cm above the floor. Five-min test sessions were performed 120 min after drug administration through intraperitoneal injection (temsirolimus and/or THC, and their vehicles). The percentage of time spent in the open arms was determined as a measure of anxiety-like processes. The model is based on rodents' aversion of open spaces. Anxiety reduction in the elevated plus maze

test is indicated by an increase in the proportion of time spent in the open arms

**Antinociception (Acetic acid test)**

This visceral pain test was performed 120 min after THC administration. Mice received an i.p. administration of acetic acid (0.8 % solution) injected in a volume of 0.1 ml/kg of body weight. Immediately after, mice were placed in individual transparent cylinders (16 cm high, 16 cm diameter) for 20 min. The number of writhes was counted during 15 min, starting 5 min after acetic acid administration.

**Results**

Temsirolimus shows amnesic effects at high doses (10, 30 and 100 mg/kg/day) (30, 90 and 300 mg/m$^2$/day). However, at a dose of 1 mg/kg/day (3 mg/m$^2$/day) it does not have effects on the recognition memory (Figure 1).

THC always presents an amnesic effect in the object-recognition test producing a cognitive effect lasting after finishing the treatment with THC.

The pre-treatment with 1 mg/kg/day (3 mg/m$^2$/day) of temsirolimus blocks the amnesic effect of 10 mg/kg/day (30 mg/m$^2$/day) THC (Figure 2). It has the same effect when both compounds are administered in combination (Figure 3).

A dose of 1 mg/kg/day (3 mg/m$^2$/day) of temsirolimus blocks the anxiogenic effects of 10 mg/kg/day (30 mg/m$^2$/day) THC (Figure 4).

Low doses of THC cause anxiolytic effects whereas high doses of the same compound cause anxiogenic effects.

A dose of 0.3 mg/kg/day (0.9 mg/m$^2$/day) of THC causes anxiolytic effects since animals spent more time in the open corridors of the V-maze. A dose of 1 mg/kg/day (3 mg/m$^2$/day) does not alter the anxiolytic effects of 0.3 mg/kg/day (0.9 mg/m$^2$/day) of THC (Figure 5).

THC has analgesic effects since mice show less abdominal writhes when they have been pre-treated with 10 mg/kg/day (30 mg/m$^2$/day) of THC. Figure 6 shows that the pre-treatment with 1mg/kg/day (3 mg/m$^2$/day) of temsirolimus and 10 mg/kg/day (30 mg/m$^2$/day) of THC does not affect the analgesic capacity of THC.

**Claims**

1. Temsirolimus for use as a medicament in the prevention and/or treatment of a Δ9-tetrahydrocannabinol-induced anxiety disorder in a mammal.

2. Temsirolimus for use according to claim 1, wherein the mammal is a human.

3. Temsirolimus for use according to claims 1 or 2, wherein the medicament is prepared for the administration of a daily dose of temsirolimus not having amnesic effects.

4. Temsirolimus for use according to any of claims 1 to 3, wherein the medicament is prepared for the administration of a daily dose of temsirolimus of from 1.5 mg/m$^2$ to 15 mg/m$^{2.}$

5. Temsirolimus for use according to claim 4, wherein the medicament is prepared for the administration of a daily dose of temsirolimus of 3 mg/m$^2$.

6. Temsirolimus for use according to any preceding claim, wherein the medicament comprises temsirolimus as sole therapeutic agent.

7. Temsirolimus for use according to claims 1 to 5, wherein the medicament comprises temsirolimus and Δ9-tetrahydrocannabinol.

8. Temsirolimus for use according to claim 7, wherein the medicament comprises temsirolimus and more than 9 mg/m$^2$/day of Δ9-tetrahydrocannabinol.

9. Temsirolimus for use according to claims 7 or 8, wherein temsirolimus and A9-tetrahydrocannabinol are contained in separate dosage forms.

10. Temsirolimus for use according to claims 7 or 8, wherein temsirolimus and Δ9-tetrahydrocannabinol are contained in a single dosage form.

**11.** Temsirolimus for use according to claims 1 to 10, wherein the Δ9-tetrahydrocannabinol is administered as a daily dose of 30 $mg/m^2$.

**12.** Temsirolimus for use according to claims 1 to 9 and 11, wherein temsirolimus is administered before the administration of Δ9-tetrahydrocannabinol.

**13.** Temsirolimus for use according to claims 1 to 11, wherein temsirolimus is administered concomitantly with Δ9-tetrahydrocannabinol.

**14.** A pharmaceutical composition comprising:

(iv) a pharmaceutically effective amount of temsirolimus,
(v) a pharmaceutically effective amount of Δ9-tetrahydrocannabinol; and
(vi) at least one pharmaceutically acceptable excipient.

**15.** Pharmaceutical composition according to claim 15 wherein the dose of temsirolimus is from 2.40 to 24.60 mg and the dose of Δ9-tetrahydrocannabinol is from 14.40 to 145.80 mg.

FIGURE 1

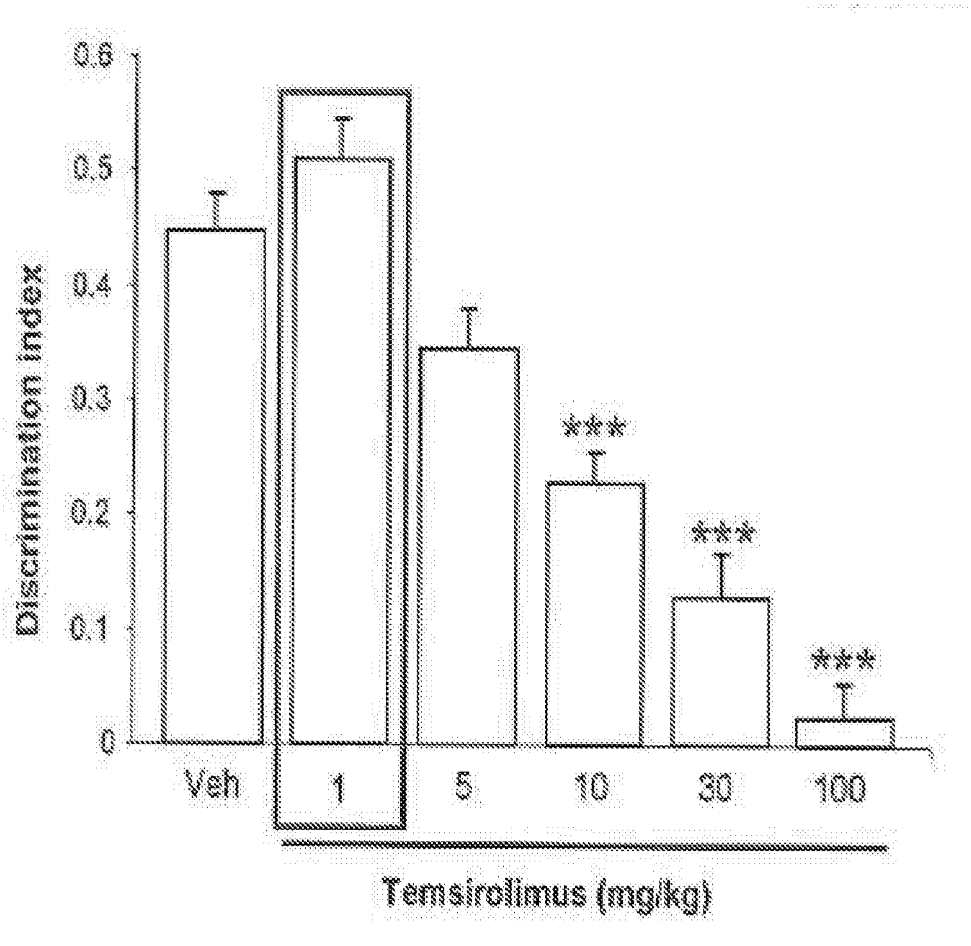
0.6
0.5
0.4
0.3
0.2
0.1
0
Discrimination index
***
***
***
Veh
1
5
10
30
100
Temsirolimus (mg/kg)

FIGURE 2

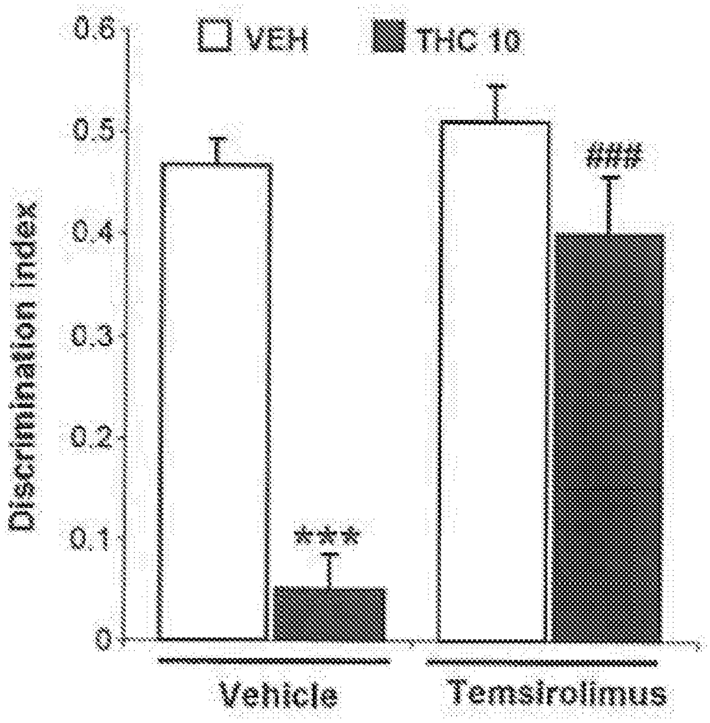
VEH
THC 10
Discrimination index
0.6
0.5
0.4
0.3
0.2
0.1
0
***
###
Vehicle
Temsirolimus

**FIGURE 3**

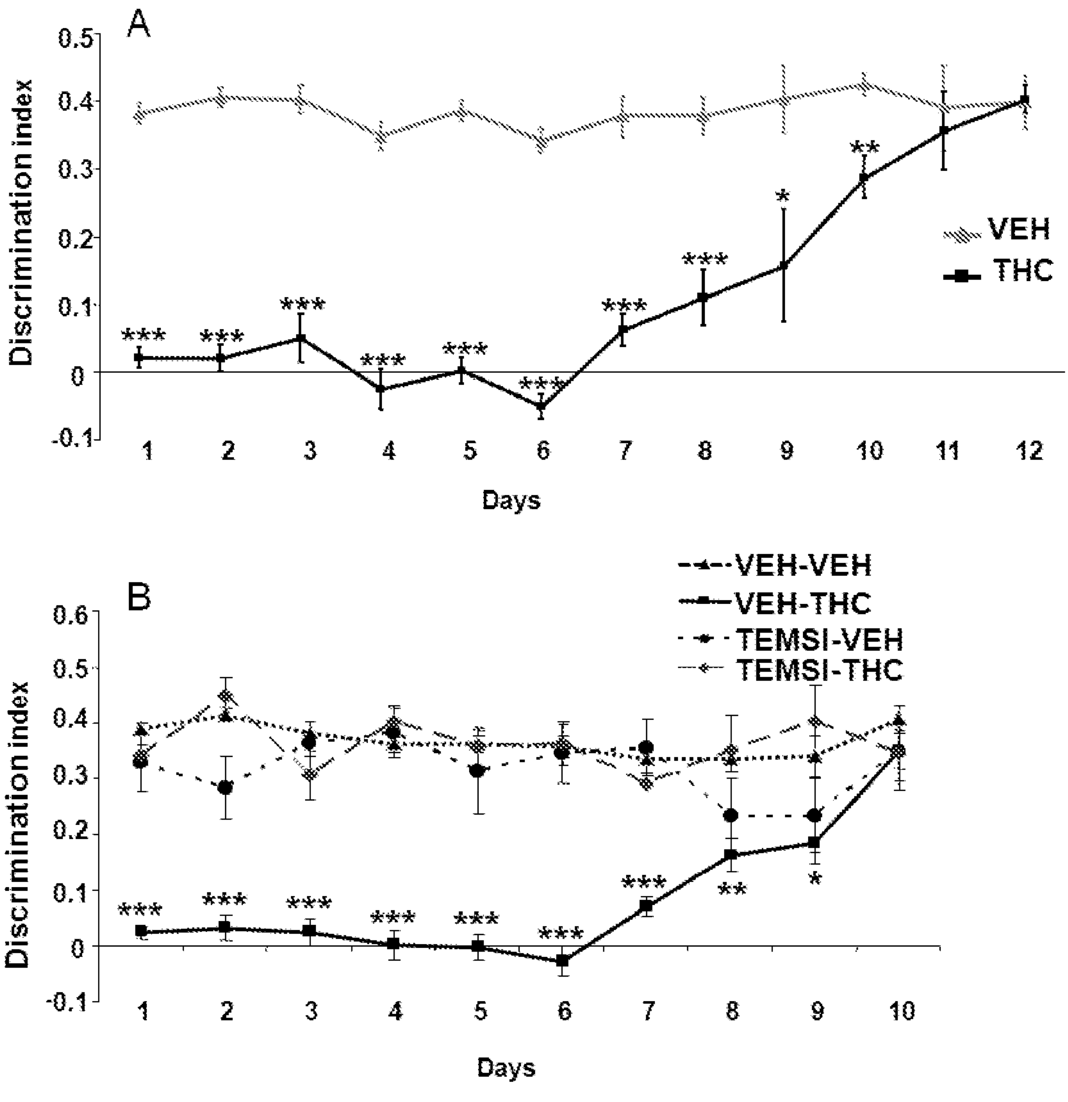
A
Discrimination index
0.5
0.4
0.3
0.2
0.1
0
-0.1
1 2 3 4 5 6 7 8 9 10 11 12
Days
VEH
THC
***
**
*
B
VEH-VEH
VEH-THC
TEMSI-VEH
TEMSI-THC
Discrimination index
0.6
0.5
0.4
0.3
0.2
0.1
0
-0.1
1 2 3 4 5 6 7 8 9 10
Days

FIGURE 4

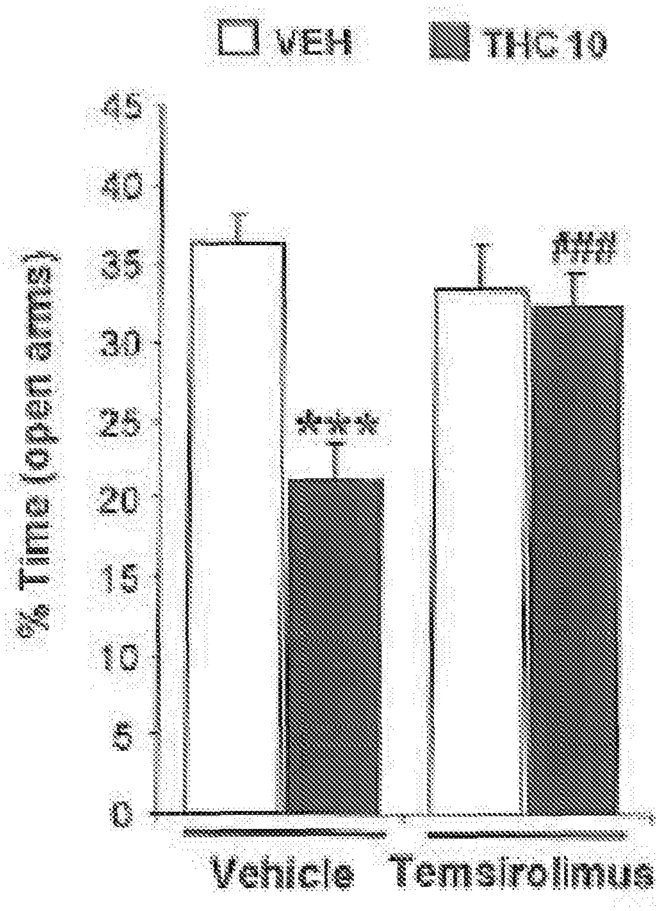
VEH
THC 10
% Time (open arms)
45
40
35
30
25
20
15
10
5
0
***
###
Vehicle
Temsirolimus

FIGURE 5

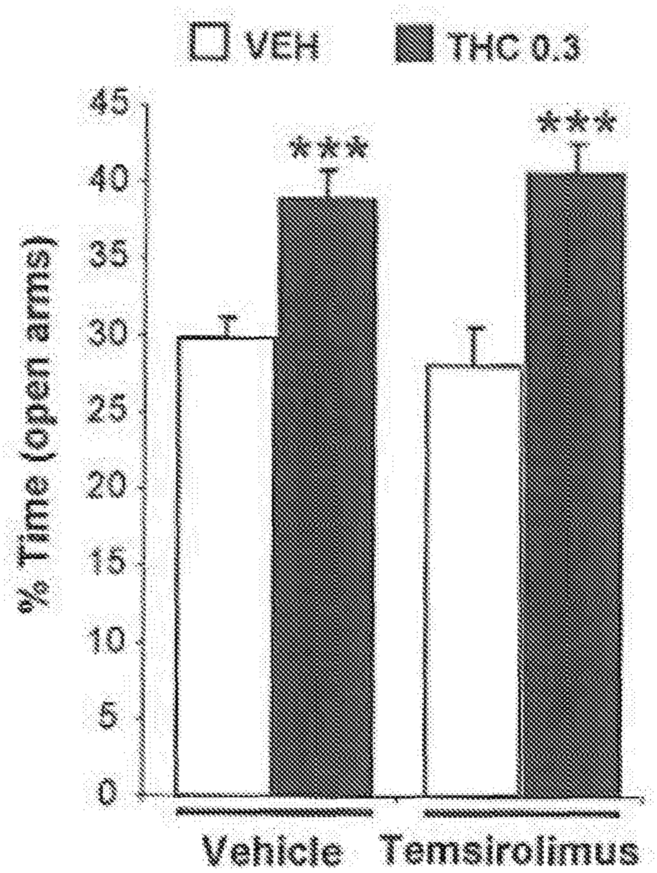
VEH
THC 0.3
% Time (open arms)
45
40
35
30
25
20
15
10
5
0
***
***
Vehicle
Temsirolimus

FIGURE 6

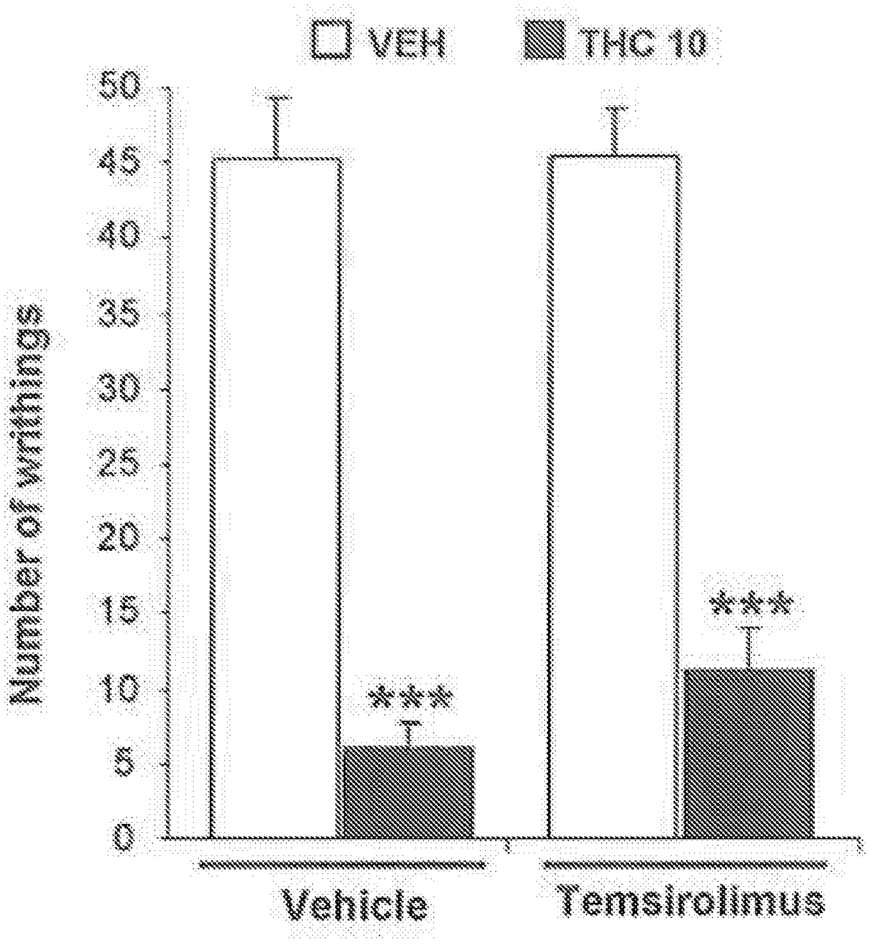
VEH
THC 10
Number of writhings
50
45
40
35
30
25
20
15
10
5
0
***
***
Vehicle
Temsirolimus

Europäisches Patentamt
European Patent Office
Office européen des brevets

# EUROPEAN SEARCH REPORT

Application Number
EP 11 17 5435

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2005/117880 A2 (UNIV COLUMBIA [US] NEW YORK THE TRUSTEES OF COLUM [US]) 15 December 2005 (2005-12-15) * claims 1,3,9 * ----- | 1-15 | INV. A61K31/355 A61K31/436 ADD. A61P25/22 |
| A | BLUNDELL J ET AL: "Systemic inhibition of mammalian target of rapamycin inhibits fear memory reconsolidation", NEUROBIOLOGY OF LEARNING AND MEMORY, SAN DIEGO, US, vol. 90, no. 1, 1 July 2008 (2008-07-01), pages 28-35, XP022708199, ISSN: 1074-7427, DOI: 10.1016/J.NLM.2007.12.004 [retrieved on 2008-03-07] * page 30, column 1, lines 25-27 * * page 34, column 1, paragraph 3 * ----- | 1-15 | |
| A | WO 2008/124125 A1 (WYETH CORP [US]; DUKART GARY [US]; GIBBONS JAMES JOSEPH JR [US]; BERKE) 16 October 2008 (2008-10-16) * the whole document * ----- | 1-15 | TECHNICAL FIELDS SEARCHED (IPC) A61K A61P |

The present search report has been drawn up for all claims

1

| Place of search | Date of completion of the search | Examiner |
|---|---|---|
| Munich | 9 September 2011 | Allnutt, Sarah |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 17 5435

This annex lists the patent family members relating to the patent documents cited in the above-mentioned European search report.
The members are as contained in the European Patent Office EDP file on
The European Patent Office is in no way liable for these particulars which are merely given for the purpose of information.

09-09-2011

| Patent document cited in search report | Publication date | Patent family member(s) | Publication date |
| --- | --- | --- | --- |
| WO 2005117880 A2 | 15-12-2005 | NONE | |
| WO 2008124125 A1 | 16-10-2008 | AU 2008236621 A1 | 16-10-2008 |
| | | CA 2681451 A1 | 16-10-2008 |
| | | CL 10092008 A1 | 17-10-2008 |
| | | CN 101678008 A | 24-03-2010 |
| | | EP 2144611 A1 | 20-01-2010 |
| | | JP 2010523660 A | 15-07-2010 |
| | | KR 20090130040 A | 17-12-2009 |
| | | PA 8776101 A1 | 31-03-2009 |
| | | US 2008255177 A1 | 16-10-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GULLETT N. et al.** *J Soc Integr Oncol,* 2009, vol. 7 (4), 155-169
- **CRIPPA J.A. et al.** *Hum Psychopharmacol,* 2009, vol. 24 (7), 515-23
- **GUILLEM E. et al.** *Encephale,* 2009, vol. 35 (3), 226-233
- **LEWEKE F.M. ; KOETHE D.** *Addict Biol,* 2008, vol. 13 (2), 264-275
- **WILLIAMSON E.M. ; EVANS F.J.** *Drugs,* 2000, vol. 60 (6), 1303-1314
- **HAY N. ; SONENBERG N.** *Genes Dev,* 2004, vol. 18 (16), 1926-45
- **BROWN H.** *Psychopharmacologia,* 1972, vol. 27 (2), 11-16
- **EUBANKS LM. et al.** *Molecular Pharmaceutics,* 2006, vol. 3 (6), 773-777
- **MOREIRA F.A. ; WOTJAK C.T.** *Curr Top Behav Neurosci,* 2010, vol. 2, 429-450
- **FAULI ; TRILLO C.** Tratado de Farmacia Galénica. 1993
- Remington: The Science and Practice of Pharmacy. Lippincott Williams & Wilkins, 2003
- **REAGAN-SHAW S.** Dose translation from animal to human studies revisited. *FASEB J,* 2007, vol. 22, 659-661
- **PUIGHERMANAL, E. et al.** *Nature Neuroscience,* 2009, vol. 12, 1152-1158